# EUROPEAN PATENT APPLICATION

(11) **EP 4 400 135 A1**
(43) Date of publication of application: **17.07.2024**
(21) Application number: 22863677.5
(22) Date of filing: 06.09.2022
(51) Int. Cl.: A61M 16/00, A61M 16/01, A61M 16/10, A61M 16/12

(54) **ANESTHESIA MACHINE**

(30) Priority: 06.09.2021 CN 202111040338
(71) Applicant: Shenzhen Mindray Bio-Medical Electronics Co., Ltd., Shenzhen, Guangdong 518057 (CN)
(72) Inventor: HUANGFU, Yong, Shenzhen, Guangdong 518057 (CN); ZHOU, Xiaoyong, Shenzhen, Guangdong 518057 (CN); CHEN, Yiwei, Shenzhen, Guangdong 518057 (CN); YANG, Shuwang, Shenzhen, Guangdong 518057 (CN); CAI, Kun, Shenzhen, Guangdong 518057 (CN)
(74) Representative: KIPA AB
(86) International application number: PCT/CN2022/117392
(87) International publication number: WO 2023/030543

(57) **Abstract**

An anesthesia machine, comprising an anesthetic delivery device (20), a breathing circuit (30), a first ventilation control device (40), and a ventilation module (50). The anesthetic delivery device (20) mixes a first gas with an anesthetic to obtain a second gas, and delivers the second gas to the breathing circuit (30). The first ventilation control device (40) controls the breathing circuit (30) to periodically deliver the second gas to a patient, thereby providing anesthesia and respiratory support for the patient. The ventilation module (50) performs periodic respiratory support on the patient by using the first gas. Therefore, according to the requirements of the clinical scenario, the corresponding ventilation support can be performed on the patient by using the anesthesia machine, thereby improving the application range of the anesthesia machine without using additional equipment.

## Description

### TECHNICAL FIELD

The disclosure relates to a technical field of medical device, and more particularly to an anesthesia machine.

### BACKGROUND

Anesthesia machines are mainly configured to provide oxygen, anesthesia, and respiratory support to patients during surgery. During the ventilation and anesthesia processes, the anesthesia machine delivers an anesthetic mixture to the patient while receiving an exhaled gas of the patient. In order to save anesthetic gas and improve a comfort degree of the patient when inhaling gas, the exhaled gas is generally recycled. In the circulatory and re-respiratory system, part of the exhaled gas of the patient is re-inhaled as an inhaled gas. In this way, the anesthetic, as a relatively expensive gas component, is retained to the greatest extent, which plays an important role in reducing environmental pollution and saving costs at the same time. Exhaled gas of the patient contains CO₂, which can cause acidosis if it is directly re-inhaled, so the CO₂ needs to be removed before recycling the exhaled gas. A common method is to use a CO₂ absorbent (soda lime) to react with CO₂ to remove CO₂. At the same time, the reaction generates water and heat, which is beneficial to maintaining the temperature and humidity of the gas inhaled by the patient.

For the above reasons, it is necessary to connect an airway system of the anesthesia machine to the respiratory system of the patient and ensure gas tightness, so as to implement effective ventilation and anesthesia. The common method is to insert a tracheal tube into trachea of the patient through endotracheal intubation, so as to connect the anesthesia machine and the trachea of the patient. At the same time, a balloon at the end of the tracheal tube can ensure the gas tightness of this connection and prevent damage to an inner wall of the trachea.

However, in some surgeries for pharyngeal, tracheal, bronchi or lungs, endotracheal intubation cannot be used because it blocks a surgical site, making the surgical operation impossible, or because this ventilation method causes the surgical site to fluctuate periodically according to inhalation and expiration of the ventilation, which affects the surgical effect. Usually when encountering surgical situations like this, additional high-frequency jet device (such as high-frequency jet ventilator) is required. Such device is rarely equipped in anesthesia operation rooms, and the frequency of adding and using the high-frequency jet device is not high, which causes problems in multi-device management. Therefore, many hospitals do not have the conditions for this kind of surgery and can only transfer to other hospitals for surgical treatment, or can only use other compromise methods, which greatly reduces the surgical effect and even brings surgical risks.

Therefore, existing anesthesia machines have limited usage scenarios.

### SUMMARY

This disclosure mainly provides an anesthesia machine, so as to improve an application scope of the anesthesia machine.

One embodiment provides an anesthesia machine, including:
a monitoring apparatus for flow amount, which is configured to regulate the flow amount of a first gas;
an anesthetic delivery apparatus, which is connected with the monitoring apparatus for flow amount, and is configured to mix the first gas with an anesthetic for obtaining a second gas, and to deliver the second gas to a respiratory loop;
the respiratory loop, which is configured to deliver the second gas to a patient;
a first ventilation control apparatus, which is configured to control the respiratory loop to deliver the second gas to the patient, so as to provide for the patient anesthesia respiratory support which is periodic; and
a human-computer interaction apparatus, which is configured to receive, from a use, a respiratory support mode which is provided for the patient by the anesthesia machine;
a ventilation module, which is configured to receive the first gas and provide for the patient respiratory support which is periodic by using the first gas;
a ventilation module, which is configured to receive a first gas and provide, by using said first gas, for said patient respiratory support which is periodic;
wherein the anesthesia machine is further configured to enable the first ventilation control apparatus to provide for said patient anesthesia respiratory support which is periodic, and/or enable the ventilation module to provide for said patient respiratory support which is periodic, according to the respiratory support mode, which is received by the human-computer interaction apparatus; wherein respiratory support which is periodic comprises no gas delivery to a patient for at least a period of time in a respiratory cycle.

One embodiment provides an anesthesia machine, including a gas source interface, an anesthetic delivery apparatus which is connected with the gas source interface, a respiratory loop, and a first ventilation control apparatus;
the gas source interface is connected with an external gas source;
the anesthetic delivery apparatus is configured to mix a gas, which is provided by the external gas source, with an anesthetic, and deliver a mixed gas into the respiratory loop;
the first ventilation control apparatus is configured to control the respiratory loop to periodically deliver the mixed gas to a patient, so as to provide anesthesia respiratory support to said patient;
wherein the anesthesia machine further includes:
   a ventilation module, which is configured to provide respiratory support to a patient by using a gas, which is provided by the external gas source; wherein the ventilation module includes a gas delivery branch, which is configured to receive said gas, which is provided by the external gas source, and provide respiratory support to said patient at a ventilation frequency of 3Hz or above.

One embodiment provides an anesthesia machine, including a gas source interface, an anesthetic delivery apparatus which is connected with the gas source interface, a respiratory loop, and a first ventilation control apparatus;
the gas source interface is connected with an external gas source;
the anesthetic delivery apparatus is configured to mix a gas, which is provided by the external gas source, with an anesthetic, and deliver a mixed gas into the respiratory loop;
the first ventilation control apparatus is configured to control the respiratory loop to periodically deliver the mixed gas to a patient, so as to provide anesthesia respiratory support to the patient;
wherein the anesthesia machine further includes:
   a ventilation module, which is connected with a jet accessory and configured to receive and regulate a gas, which is outputted from the gas source interface, so as to enable said gas to provide respiratory support to a patient in a jet gas flow after passing through the jet accessory.

One embodiment provides a ventilation control method for an anesthesia machine, including receiving a first gas; receiving, from a user, a respiratory support mode which is provided by the anesthesia machine; when the received respiratory support mode is anesthesia respiratory support which is periodic, controlling the first gas to mix with an anesthetic, so as to obtain a second gas, controlling the second gas to enter into a respiratory loop of the anesthesia machine, and controlling the respiratory loop to periodically output the second gas, so as to provide anesthesia respiratory support which is periodic; when the received respiratory support mode is respiratory support which is periodic, controlling the first gas to enter into a ventilation module of the anesthesia machine, and controlling the ventilation module to periodically output the first gas, so as to provide respiratory support which is periodic; wherein respiratory support which is periodic includes no gas delivery for at least a period of time in a respiratory cycle.

The anesthesia machine according to above embodiments includes an anesthetic delivery apparatus, a respiratory loop, a first ventilation control apparatus and a ventilation module; wherein the anesthetic delivery apparatus mixes the first gas with the anesthetic and delivers the second gas into the respiratory loop, the first ventilation control apparatus controls the respiratory loop to periodically deliver the second gas to the patient, thereby providing anesthesia respiratory support to the patient. The ventilation module uses the first gas to provide respiratory support which is periodic to the patient. Therefore, the anesthesia machine can provide corresponding ventilation support to the patient according to requirements of clinical scenarios, which improves the application range of the anesthesia machine without requiring an additional device.

### BRIEF DESCRIPTION OF THE DRAWINGS

FIG. 1 is a structural block diagram of an existing anesthesia machine.
FIG. 2 is a structural block diagram of an embodiment of an anesthesia machine provided by this disclosure.
FIG. 3 is a structural block diagram of an embodiment of an anesthesia machine provided by this disclosure.
FIG. 4 is a structural block diagram of an embodiment of an anesthesia machine provided by this disclosure.
FIG. 5 is a structural block diagram of an embodiment of a ventilation module in an anesthesia machine provided by this disclosure.
FIG. 6 is a gas path diagram of an embodiment of a ventilation module in an anesthesia machine provided by this disclosure.
FIG. 7 is a gas path diagram of an embodiment of a ventilation module in an anesthesia machine provided by this disclosure.
FIG. 8 is a gas path diagram of an embodiment of a ventilation module in an anesthesia machine provided by this disclosure.
FIG. 9 is a pressure waveform diagram displayed on a display in an anesthesia machine provided by this disclosure.
FIG. 10 is a diagram of a main monitoring interface on a display in an anesthesia machine provided by this disclosure.
FIG. 11 is a structural block diagram of a monitoring apparatus for flow amount in an embodiment shown in FIG. 3.
FIG. 12 is a structural block diagram of a monitoring apparatus for flow amount in an embodiment shown in FIG. 4.
FIG. 13 is a gas path diagram of an embodiment of a jet apparatus in an anesthesia machine provided by this disclosure.

### DESCRIPTION OF THE PREFERRED EMBODIMENTS

This disclosure is further described in detail below through specific embodiments in combination with the accompanying drawings. In different embodiments, similar elements adopt associated similar reference numbers. In the following embodiments, many details are described in order to make this disclosure better understood. However, one skilled in the art can easily recognize that some of the features can be omitted in different cases, or can be replaced by other elements, materials and methods. In some cases, some operations related to this disclosure are not shown or described in the description, in order to avoid the core part of this disclosure being inundated by too many descriptions. For one skilled in the art, it is not necessary to describe these related operations in detail, as they can fully understand the relevant operations according to the description in this disclosure and the general technical knowledge in the art.

In addition, the features, operations, or characteristics described in the specification may be combined in any appropriate manner to form various embodiments. At the same time, the steps or actions in the method description can also be exchanged or regulated in order in a manner obvious to one skilled in the art. Therefore, the various sequences in the description and the drawings are only for the purpose of clearly describing a certain embodiment, and do not mean that they are necessary sequences. Unless it is otherwise specified that one of the sequences must be followed.

The terms "first", "second", etc. in the specification are operable to distinguish different objects, rather than to describe a specific order. In addition, the terms "connection", and "linkage" mentioned in this disclosure include direct and indirect connection (linkage), unless otherwise specified.

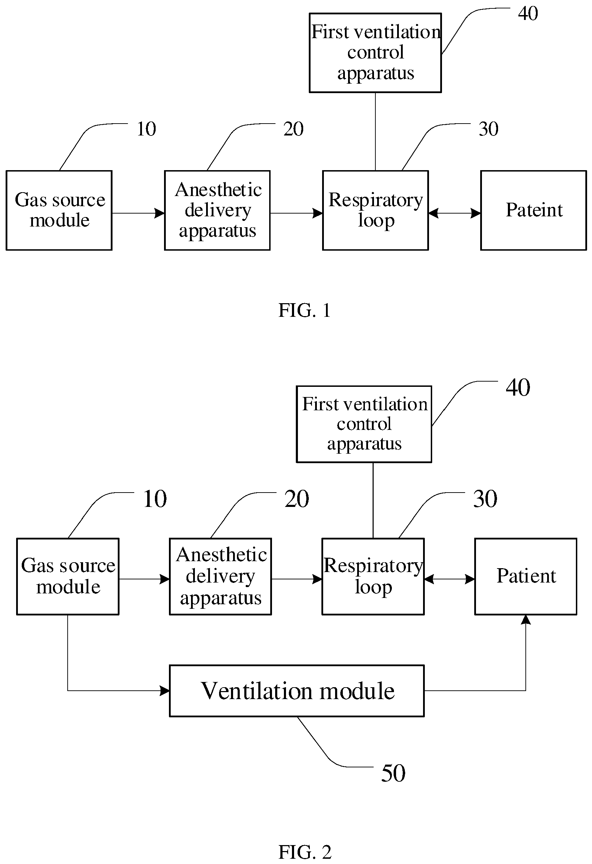

As shown in FIG. 1, an existing anesthesia machine generally includes a gas source module 10, an anesthetic delivery apparatus 20, a respiratory loop 30 and a first ventilation control apparatus 40.

The gas source module 10 is connected with the respiratory loop 30 through the anesthetic delivery apparatus 20, that is, the gas source module 10, the respiratory loop 30 and the anesthetic delivery apparatus 2 are connected in sequence in a gas path.

The gas source module 10 is configured to provide a gas which is required by the anesthesia machine, that is, to provide a first gas, such as one or more of oxygen, air and laughing gas (nitrous oxide), because it is necessary to provide respiratory support to the patient. Therefore, the gas provided contains at least oxygen. That is, the first gas may be oxygen, air, an oxygen-air mixed gas, or a mixed gas of oxygen and laughing gas. The gas source module 10 may be a gas source interface which is connected with an external gas source. The external gas source provides the gas required by the anesthesia machine to the anesthesia machine through the gas source interface. The gas source module 10 can also be an air compressor, an oxygen generator, etc., and generate the first gas, which is required by the anesthesia machine by itself. Subsequent embodiments are described by using the gas source interface as an example.

The anesthetic delivery apparatus 20 is configured to mix the first gas, which is provided by the gas source module 10, with an anesthetic, control an anesthetic concentration of the mixed gas (second gas), and deliver the mixed gas (second gas) to the respiratory loop 30. The anesthetic delivery apparatus 20 includes a vaporizer.

The respiratory loop 30 is a gas path, which connects the anesthetic delivery apparatus 20 and the patient, and can recycle an exhaled gas of the patient to save anesthetic and reduce environmental pollution. The respiratory loop 30 can include various connection tubes and accessories. The accessories can be an endotracheal tube, an endotracheal tube with a balloon at its end, etc. A gas purification apparatus may be provided in the respiratory loop 30, and configured to remove at least part of carbon dioxide exhaled by the patient into the respiratory loop. For example, CO₂ absorbent (soda lime) can be installed in the gas purification apparatus. The CO₂ absorbent reacts with CO₂ to achieve the purpose of removing CO₂. At the same time, the reaction generates water and heat, which is beneficial to maintaining the temperature and humidity of inhaled gas the patient.

The first ventilation control apparatus (i.e., an anesthesia ventilation control apparatus) 40 is configured to control the respiratory loop 30 to periodically deliver the mixed gas (second gas) to the patient, thereby providing anesthesia respiratory support which is periodic to the patient. The first ventilation control apparatus 40 can control anesthesia ventilation automatically or manually (such as a balloon). For example, the first ventilation control apparatus 40 may include a plurality of valves and a board card for driving the plurality of valves. The board card controls the plurality of valves to periodically deliver the second gas to the patient, thereby providing anesthesia respiratory support which is periodic to the patient.

During the operation of the anesthesia machine, the first gas is provided by the gas source module 10, and gas components are mixed through the monitoring apparatus for flow amount, then an addition and concentration adjustment of the anesthetic are implemented by an evaporator, so as to form a second gas which enters into the respiratory loop 30. The first ventilation control apparatus 40 performs a ventilation control and delivers the second gas to the patient. The exhaust gas exhaled by the patient is purified by the gas purification apparatus and then discharged or recycled. During the above process, the anesthesia machine also monitors a machine state and patient parameter(s) to ensure patient safety and send out abnormal alarm.

This disclosure creatively integrates a ventilation module which provides respiratory support which is periodic on an anesthesia machine, so as to enable the anesthesia machine to provide respiratory support which is periodic through the ventilation module and/or to provide anesthesia respiratory support which is periodic through the first ventilation control apparatus. For example, in some specific scenarios, such as when the inhalation anesthesia function of the anesthesia machine does not need to be used, the ventilation module of the anesthesia machine can be configured to provide for the patient respiratory support, which improves the application scope of the anesthesia machine, eliminates the need to use the additional device, saves management issue of multiple devices. Wherein, respiratory support which is periodic provided by the ventilation module can be high-frequency ventilation or low-frequency ventilation, etc. For example, the ventilation module includes a gas delivery branch, which receives the gas provided by the external gas source and provides respiratory support to the patient at a ventilation frequency of 3 Hz or above). The ventilation mode of the ventilation module can be jet ventilation or non-jet ventilation. When providing the jet ventilation, the ventilation module can further provide high-frequency jet ventilation. Detailed description is given below through some examples.

As shown in FIG. 2, the anesthesia machine provided by this disclosure adds a ventilation module 50, which is connected with the gas source module 10 on the basis of the existing anesthesia machine. The gas source module 10 and the ventilation module 50 can be in direct connection or indirect connection, in short, just in connection. The ventilation module 50 is configured to provide respiratory support which is periodic to the patient, by using the first gas (such as oxygen and/or air) provided by the gas source module 10. It can be seen that, when no inhalation anesthesia is required, the anesthesia machine can also be configured to ventilate the patient, which improves the application scope of the anesthesia machine without requiring additional device(s). In some usage scenarios, the human-computer interaction apparatus provided by the anesthesia machine can be configured to receive a respiratory support mode, which is provided for the patient by the anesthesia machine. For example, trigger button(s) for different respiratory support modes can be provided on a display interface of the human-computer interaction apparatus. The anesthesia machine can decide, based on the respiratory support mode received by the human-computer interaction apparatus, whether to use the ventilation module to provide respiratory support which is periodic to the patient, or to use the first ventilation control apparatus to control the respiratory loop to provide anesthesia respiratory support which is periodic to the patient; or to simultaneously use both of the ventilation module and the first ventilation control apparatus to provide both respiratory supports to the patient. "Respiratory support which is periodic" and "anesthesia respiratory support which is periodic" mentioned in this disclosure represent two different modes of respiratory support. What they have in common is that they do not provide gas delivery to the patient for at least a period of time in a respiratory cycle, what they have in difference is that anesthesia respiratory support which is periodic includes the anesthetic in its delivered gas, while respiratory support which is periodic does not include the anesthetic in its delivered gas.

The ventilation module 50 can be connected with various accessories, such as a jet accessory, a nasal plug, a nasal mask, a mask, etc. The doctor can connect an appropriate accessory and perform corresponding ventilation method according to actual condition of the patient and ventilation requirements, which is very convenient.

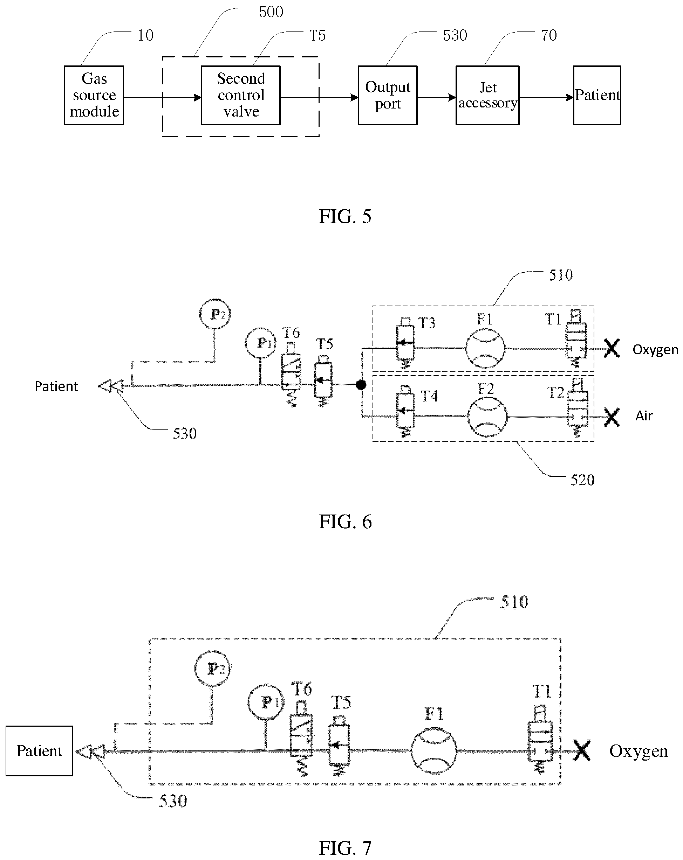

The ventilation module 50 can be implemented in a variety of ways, as shown in FIGS. 6, 7 and 8. In provided respiratory support which is periodic, the specific ventilation method can be high-frequency ventilation and/or jet ventilation, wherein the jet ventilation can be high-frequency jet ventilation, or low-frequency jet ventilation. The expression "high frequency/jet ventilation" as used herein refers to either high frequency ventilation or jet ventilation. Below are some examples to illustrate.

### First Embodiment

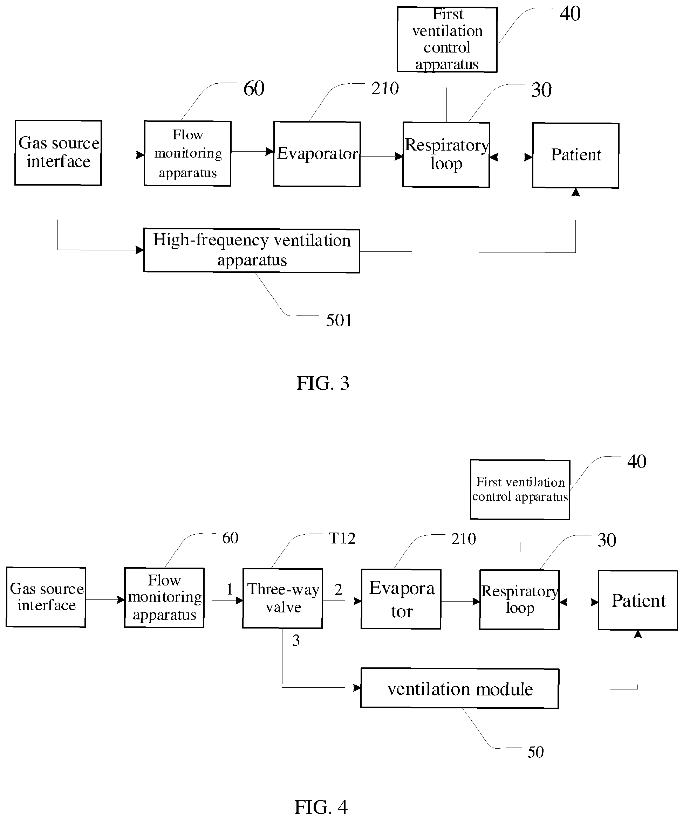

In this embodiment, the ventilation mode provided by the ventilation module 50 is high-frequency ventilation. For example, as shown in FIGS. 3 and 4, the ventilation module 50 includes a high-frequency ventilation apparatus 501. The high-frequency ventilation apparatus 501 is configured to ventilate the patient at a ventilation frequency of at least 3 Hz, thereby providing respiratory support which is periodic to the patient. As shown in FIG. 5, the high-frequency ventilation apparatus 501 may include a gas delivery branch 500, a second control valve T5 and an output port 530. The high-frequency ventilation apparatus 501 is configured to receive the first gas through the gas delivery branch 500. The second control valve T5 is arranged inside the gas delivery branch 500 and is controlled to switch an amount of flow at a switching frequency of at least 3 Hz, so that the first gas outputted from the output port 530 forms a high-frequency gas flow. The expression "high-frequency gas flow" used in this disclosure refers to gas whose output frequency is higher than a certain critical value, for example, gas whose output frequency is higher than or equal to 3 Hz.

The ventilation frequency (switching frequency of flow or switching frequency of opening degree for the valve) of the second control valve T5 can be preset. The second control valve T5 is used for high-frequency ventilation control (for example, switching between two different valve flow amounts in a high frequency which is above 3 Hz, for example, between 3-20Hz; wherein one of the two different valve flow amounts is high, while the other is low), so as to enable the first gas to form a gas flow of high-frequency pulse, which is capable of providing to the patient high-frequency jet ventilation after passing through a subsequent jet accessory, or providing to the patient high-frequency ventilation without subsequently connecting a jet accessory. The second control valve T5 may be an electromagnetic valve, an electromagnetic servo valve, an electromagnetic switch valve or an electromagnetic proportional valve, etc.

In this embodiment, the high-frequency ventilation apparatus 501 provides respiratory support which is periodic at a first ventilation frequency, and the first ventilation control apparatus 40 controls the respiratory loop 30 to provide anesthesia respiratory support which is periodic at a third ventilation frequency. The first ventilation frequency is higher than the third ventilation frequency. For example, the first ventilation frequency is higher than or equal to 3 Hz, and the third ventilation frequency is less than 3 Hz.

The high-frequency ventilation apparatus 501 can be implemented in various ways, as shown in FIG. 6, FIG. 7, and FIG. 8, which are introduced one by one below.

As shown in FIG. 7, the first gas may include oxygen. The gas source module 10 includes an oxygen unit which provides oxygen. For example, the oxygen unit is an oxygen interface or an oxygen generator. The oxygen interface is connected with an external oxygen source. For example, the oxygen interface is connected with an oxygen delivery pipeline, an oxygen bottles, in the hospital, etc. The gas delivery branch 500 includes an oxygen branch 510, that is, the ventilation module 50 or the high-frequency ventilation apparatus 501 includes: the oxygen branch 510, the second control valve T5 and the output port 530. The oxygen branch 510 is connected with the oxygen unit, for example, the oxygen branch 510 and the oxygen unit are directly connected. The oxygen unit is connected with the output port 530 through the oxygen branch 510. The second control valve T5 is arranged inside the oxygen branch 510. The second control valve T5 switches an amount of flow at a preset frequency (for example, at least 3 Hz), which is capable of controlling a flow amount of oxygen inside the oxygen branch 510, enabling the oxygen outputted by the output port 530 to form a high-frequency gas flow, thereby providing periodic high-frequency ventilation to the patient. The oxygen branch 510 may also be provided with a first switch valve T1, so as to control an on/off state of the oxygen branch 510.

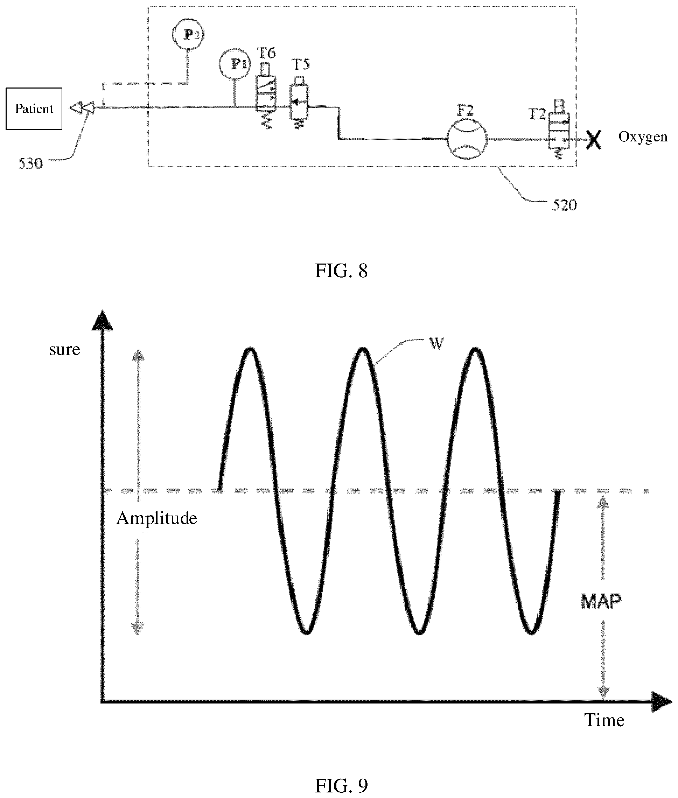

As shown in FIG. 8, the first gas may also include air. The gas source module 10 includes an air unit, which provides air. For example, the air unit is an air interface, which is connected with an external gas source, such as an air delivery duct in hospital. For another example, the air unit is a turbine, which obtains air from external atmospheric environment. The gas delivery branch 500 includes an air branch 520 , that is, the ventilation module 50 or the high-frequency ventilation apparatus 501 includes: the air branch 520, the second control valve T5 and the output port 530. The air branch 520 is connected with the air unit, for example, the air branch 520 and the air unit are directly connected. The air unit is connected with the output port 530 through the air branch 520. The second control valve T5 is arranged inside the air branch 520. The second control valve T5 switches an amount of flow at a preset frequency (for example, at least 3 Hz), which is capable of controlling a flow amount of air inside the air branch 520, enabling the air outputted by the output port 530 to form a high-frequency gas flow, thereby providing periodic high-frequency ventilation to the patient. The air branch 520 may also be provided with a second switch valve T2, so as to control an on/off state of the air branch 520.

In the embodiment shown in FIGS. 7 and 8, the second control valve T2 switches the flow amounts at a frequency that can realize high-frequency ventilation. In the process of high-frequency regulation for opening degrees of the valve, flow amounts inside the oxygen branch and the air branch are controlled by the opening degree, and the ventilation frequencies of gases in the oxygen branch and the air branch can be controlled through the switching frequency.

As shown in FIG. 6, the first gas may also include oxygen and air. The gas source module 10 includes an oxygen unit which provides oxygen, and an air unit. The gas delivery branch 500 includes an oxygen branch 510 and an air branch 520, that is, the ventilation module 50 or the high-frequency ventilation apparatus 501 includes: the oxygen branch 510, the air branch 520, the second control valve T5 and the output port 530. The oxygen branch 510 is connected with the oxygen unit, for example, the oxygen branch 510 and the oxygen unit are directly connected. The air branch 520 is connected with the air unit, for example, the air branch 520 and the air unit are directly connected. The oxygen branch 510 and the air branch 520 are converged with each other, and then connected with the output port 530 through the second control valve T5. The oxygen branch 510 is provided with a first control valve T3, which is configured to control the flow amount of the oxygen inside the oxygen branch 510. The oxygen branch 510 can also be provided with a first switch valve T1 which is configured to control an on/off state of the oxygen branch 510, wherein he first switch valve T1 plays a safety protection role. The air branch 520 is provided with a third control valve T4, which is configured to control the flow amount of the air inside the air branch 520. The air branch 520 may also be provided with a second switch valve T2 which is configured to control an on/off state of the air branch 520, wherein the second switch valve T2 plays a safety protection role. The first control valve T3 may be a proportional valve or a flow valve, and the third control valve T4 may be a proportional valve or a flow valve. The first control valve T3 and the third control valve T4 can jointly control a total flow amount and an oxygen concentration of the oxygen-air mixed gas. The second control valve T5 switches an amount of flow at a preset frequency (for example, at least 3Hz), so as to form a high-frequency gas flow from the converged oxygen-air mixed gas. The high-frequency gas flow is outputted through the output port 530, so as to provide for the patient high-frequency ventilation. In some embodiments, as an alternative to the embodiment shown in FIG. 6, the second control valve T5 may not be provided, but the first control valve T3 and the third control valve T4 may jointly realize the high-frequency ventilation control. That is, the first control valve T3 is used both to control the flow amount of the oxygen inside the oxygen branch 510, and to enable the oxygen inside the oxygen branch to form a high-frequency gas flow, thereby realizing the high-frequency ventilation. The third control valve T4 is used both to control the flow amount of the air inside the air branch 520, and to enable the air inside the air branch 520 to form a high-frequency gas flow, thereby realizing the high-frequency ventilation. In this alternative embodiment, the first control valve T3 and the third control valve T4 can switch an amount of flow at a high-frequency ventilation frequency; can control flow amounts of the gases inside the air branch and the branch oxygen according to the opening degrees of the valves, during the process of high-frequency regulation for opening degrees of the valves; and can control ventilation frequencies of gases in the oxygen branch and the air branch through the switching frequency. Therefore, as an alternative to the embodiment shown in FIG. 6, by controlling the first control valve T3 and the third control valve T4 to switch an amount of flow at a high-frequency ventilation frequency (such as greater than or equal to 3Hz), such as switching between an on state or a partial state off at a high-frequency, the oxygen-air mixed gas forms a high-frequency gas flow, thereby providing high-frequency ventilation to the patient.

In the embodiment of FIGS. 6 to 8, both the first switch valve T1 and the second switch valve T2 play a safety protection role. When the ventilation module 50 is not in use, if the ventilation module 50 has a gas output, a gas supply of the anesthesia delivery system of the original anesthesia machine and ventilation of the anesthesia ventilator are affected, and meanwhile, losses of oxygen or gas source are also caused. Therefore, the first switch valve T1 and the second switch valve T2 are provided to prevent gas output, when the oxygen control valve T3, the air control valve T4 or the second control valve T5 cannot be closed during a time in which the ventilation module is not in use.

It can be known from the above-mentioned high-frequency ventilation apparatus 501 that the high-frequency gas flow formed at its output port 530 ventilates the patient at a ventilation frequency of at least 3 Hz. Or in some embodiments, the high-frequency gas flow formed at its output port 530 ventilates the patient in a ventilation frequency at one value between 50-1500 bpm. High-frequency ventilation includes high-frequency positive pressure ventilation, high-frequency oscillation ventilation and high-frequency jet ventilation. When the above-mentioned high-frequency ventilation apparatus is used for high-frequency positive pressure ventilation or high-frequency oscillation ventilation, the ventilation frequency for the patient is at least 3Hz. When the high-frequency ventilation apparatus is used for high-frequency jet ventilation, the ventilation frequency for the patient is at least 50-1500bpm. The high-frequency ventilation provided by the ventilation module 50 can be pure high-frequency ventilation, or high-frequency jet ventilation after adding the jet accessory 70. Specifically, if the output port 530 is not connected with the jet accessory, but to other ventilation accessories (Such as trachea, intubation, mask, nasal mask, etc.), the high-frequency ventilation apparatus 501 simply provides high-frequency ventilation for the patient. If the output port 530 is connected with the jet accessory 70, the high-frequency ventilation apparatus 501 can further provide high-frequency jet ventilation for the patient. Taking the latter as an example, the output port 530 can be connected with a jet accessory, and the high-frequency gas flow, which is outputted by the output port 530, is delivered through the jet accessory to form a gas flow of high-frequency jet. The jet accessory can be provided by a third-party manufacturer, and the user can connect it to the output port 530. Of course, the jet accessory can also a part of anesthesia machine accessories, that is, the anesthesia machine includes a jet accessory which is connected with the high-frequency ventilation apparatus 501. The jet accessory receives the first gas outputted by the high-frequency ventilation apparatus 501 and outputs the first gas in the form of a gas flow of high-frequency jet. That is, the output port 530 outputs a high-frequency gas flow (gas flow of high-frequency pulse), which becomes a gas flow of high-frequency jet after passing through the jet accessory. The ventilation frequency of the gas flow of high-frequency jet for ventilating the patient can be 3 Hz or more, or between 50-1500bpm, thus providing the patient with high-frequency jet ventilation. For high-frequency oscillation ventilation and high-frequency positive pressure ventilation, the ventilation frequency can be 3Hz or above. For high-frequency jet ventilation, the ventilation frequency can be one value between 50-1500bpm. The jet accessory includes a jet channel, an output aperture of the jet channel is less than 4mm. The jet accessory may include a jet nozzle, a jet needle, or a stent (such as a tracheoscope, a bronchoscope, a scope sheath, etc.). The stent is configured to support human tissue or connect the endotracheal tube to facilitate the surgery of the doctor. A wall of the stent is provided with a hole for the gas jet. (For example, its aperture is less than 4mm).

### Second Embodiment

In this embodiment, the ventilation mode provided by the ventilation module 50 is jet ventilation. The ventilation module 50 is further connected with a jet accessory, and is configured to receive the gas, which is outputted from the gas source interface and regulate the gas, so as to enable the gas to provide respiratory support to the patient in a jet gas flow after passing through the jet accessory. The gas path diagram is still shown in FIGS. 6-8. That is, the ventilation module 50 includes a gas delivery branch 500, a second control valve T5 and an output port 530. The second control valve T5 is arranged inside the gas delivery branch 500 and is configured to control the ventilation module 50, so as to provide respiratory support which is periodic. The output port 530 is connected with the jet accessory. The ventilation module 50 receives the first gas through the gas delivery branch 500, and the first gas is outputted through the jet accessory 70, which is connected with the output port 530, so as to form a jet gas flow. In some embodiments, the anesthesia machine may include a jet accessory 70 which is connected with the ventilation module 50. The jet accessory 70 receives the first gas, which is outputted by the ventilation module 50 and forms a jet gas flow from the first gas. In other embodiments, the anesthesia machine may not include a jet accessory, and the user can install the jet accessory to the output port 530 of the ventilation module by himself during subsequent use. The expression "jet ventilation" used in this disclosure refers to a following ventilation mode. During a gas delivery phase of one respiratory cycle, a high-speed gas is ejected into a respiratory tract by passing a high-pressure gas through a fine-aperture jet accessor, wherein the high-speed gas uses an entrainment/Venturi effect to supplement the tidal volume, and the gas delivery is stopped during an expiratory phase of one respiratory cycle. In this disclosure, the term "jet gas flow", which corresponds to jet ventilation, refers to the high-speed gas flow, which is outputted by the jet accessory.

In this embodiment, the ventilation module 50 can have various gas path diagrams, as shown in FIGS. 6, 7 and 8, which are introduced one by one below.

As shown in FIG. 7, the gas source module 10 includes an oxygen unit, and the first gas includes oxygen, which is provided by the oxygen unit. The gas delivery branch 500 includes an oxygen branch 510, that is, the ventilation module includes: an oxygen branch 510, a second control valve T5 and an output port 530. The oxygen unit is connected with the output port 530 through the oxygen branch, and the output port 530 is connected with the jet accessory. The second control valve T5 is arranged inside the oxygen branch 510, and is configured to control a flow amount of oxygen inside the oxygen branch 510 and enable the oxygen, which is outputted from the output port 530, to form a periodic jet gas flow after passing through the jet accessory, thereby providing periodic jet ventilation to the patient. Similarly, the oxygen branch 510 may also be provided with a first switch valve T1, so as to control an on/off state of the oxygen branch 510.

As shown in FIG. 8, the gas source module 10 includes an air unit, and the first gas includes air, which is provided by the air unit. The gas delivery branch 500 includes an air branch 520, that is, the ventilation module 50 includes: an air branch 520, a second control valve T5 and an output port 53. The air unit is connected with the output port 530 through the air branch 520, and the output port is connected with the jet accessory. The second control valve T5 is arranged inside the air branch 520 and is configured to control a flow amount of air inside the air branch 520 and enable the air, which is outputted from the output port 530, to form a periodic jet gas flow after passing through the jet accessory, thereby providing periodic jet ventilation to the patient. Similarly, the air branch 520 may also be provided with a second switch valve T2 so as to control an on/off state of the air branch 520.

In the embodiments of FIGS. 7 and 8, the second control valve T5 switches an amount of flow at a certain frequency, and controls flow amounts of the gases inside the air branch and the branch oxygen according to the opening degrees of the valves, during the process of high-frequency regulation for opening degrees of the valves, and controls ventilation frequencies of gases in the oxygen branch and the air branch through a switching frequency, so as to enable the oxygen branch and the air branch to respectively outputs periodic gas flows. When the periodic gas flows from the output port are outputted through the jet channel of the jet accessory, periodic injection ventilation is provided for the patient.

As shown in FIG. 6 , the gas source module 10 includes an oxygen unit which provides oxygen and an air unit which provides air. The first gas includes oxygen provided by the oxygen unit and air provided by the air unit. The gas delivery branch 500 includes an oxygen branch and an air branch, that is, the ventilation module 50 includes: an oxygen branch 510, an air branch 520, a second control valve T5 and an output port 530. The oxygen branch 510 is connected with the oxygen unit, and the air branch 520 is connected with the air unit. The oxygen branch 510 and the air branch 520 are converged with each other, and then connected with the output port 530 through the second control valve T5. The output port 530 is connected with a jet accessory. Wherein the oxygen branch 510 is provided with a first control valve T3, and the air branch 520 is provided with a third control valve T4. The first control valve T3 is configured to control the flow amount of the oxygen inside the oxygen branch 510 , and the third control valve T4 is configured to control the flow amount of air inside the air branch 520. The second control valve T5 is configured to form a periodic jet gas flow, when the converged oxygen-air mixed gas is outputted from the jet accessory, which is connected with the output port 530, thereby providing periodic jet ventilation for the patient.

In some embodiments, as an alternative to the embodiment shown in FIG. 6 , the second control valve T5 may not be provided, but the first control valve T3 and the third control valve T4 may jointly realize the jet control. That is, the first control valve T3 is used both to control the flow amount of the oxygen inside the oxygen branch 510, and to enable the oxygen inside the oxygen branch to form a periodically outputted oxygen flow, so as to realize the jet ventilation. The third control valve T4 is used both to control the flow amount of the air inside the air branch 520, and to enable the air inside the air branch to form a periodically outputted air flow, so as to realize the jet ventilation. That is, when the mixed gas, which is formed by the periodic oxygen flow of the oxygen branch 510 and the periodic air flow of the air branch 520, is outputted from the jet accessory, which is connected with the output port 530, a periodic jet gas flow is formed.

It can be seen from the embodiments in FIGS. 6-8 that, in this embodiment, the ventilation module 50 provides respiratory support which is periodic to the patient by performing jet ventilation on the patient. Jet ventilation is one of positive pressure ventilation. Jet ventilation is a ventilation manner that ejects high-pressure gas into the respiratory tract at high speed under an open airway. Jet ventilation has a wide range of ventilation frequencies. In order to satisfy the requirements of user with different physiological conditions, jet ventilation can have a variety of ventilation frequencies, such as high-frequency jet ventilation and low-frequency jet ventilation. High frequency and low frequency are relative concepts, which are equivalent to multiple ventilation frequency gears. The frequency of high-frequency jet ventilation is higher than the frequency of low-frequency jet ventilation. In some embodiments, for example, the frequency of high-frequency jet ventilation can be between 50 and 1500 bpm. In other embodiments, the frequency of high-frequency jet ventilation can be above 3 Hz, such as between 3 and 20 Hz. The frequency of low-frequency ventilation can be, for example, between 10-50 bpm. In this disclosure, another difference between jet ventilation and anesthesia respiratory support which is periodic and controlled by the first ventilation control apparatus, is that the jet ventilation is positive pressure ventilation under an open airway, while anesthesia respiratory support which is periodic, is applied to a ventilation scenarios of closed airway closure or intubation.

In the existing technology, some anesthesia machines, in addition to providing anesthesia respiratory support to patients, can also provide an oxygen therapy of high-flow amount, that is, deliver oxygen of high-flow amount to the patient, thereby achieving pre-oxygenation of the patient and extending asphyxiation time window of the patient, leaving time for doctors to perform intubation and other operations. Pre-oxygenation does not involve the control of the respiratory cycle, but the newly added ventilation module 50 in this embodiment needs to provide respiratory support which is periodic to the patient, which involves the control of the respiratory cycle, that is, involves the control of the ventilation frequency.

In one embodiment, the ventilation module 50 may include a second ventilation control apparatus. For example, the second ventilation control apparatus may also include one or more valves and a board card for driving the one or more valves. The one or more valves are provided inside the gas delivery branch (such as the above-mentioned oxygen branch, air branch, etc). The board card controls one or more valves (such as various control valves as shown in FIGS. 6-8) to periodically deliver the first gas to the patient, thereby providing respiratory support which is periodic to the patient. The board card controls an opening degree of one or more valves at a certain frequency, so as to form a periodic gas flow, so that the ventilation module 50 can provide respiratory support which is periodic to the patient. Combined with the above embodiments, when the second ventilation control apparatus controls the switching frequencies of the opening degrees of the one or more valves to be above 3 Hz, the ventilation module 50 can provide high-frequency ventilation for the patient. When the second ventilation control apparatus controls the switching frequencies of the opening degrees of the one or more valves to be 10-1500 bpm, and the ventilation module 50 is connected with a jet accessory, the ventilation module 50 can provide jet ventilation to the patient.

In the above embodiment, the oxygen branch 510 may also be provided with a first flow sensor F1 for monitoring the flow amount of the oxygen branch 510. The air branch 520 may also be provided with a first flow sensor F2 for monitoring the flow amount of the air branch 520. Through these two flow sensors, the flow amounts of oxygen and air can be well monitored.

A safety valve T6 for pressure relief, and at least one pressure sensor for monitoring pressure, are provided between a converging node (black dot in FIG. 6) of the oxygen branch 510 and the air branch 520, and the output port 530. The safety valve T6 can be specifically configured to connect the output port 530 with an exhaust end of the safety valve T6, so as to discharge the gas between the output port 530 and the safety valve T6 through the exhaust end for preventing the patient from barotrauma, when a pressure monitored by the pressure sensor exceeds a preset threshold. The safety valve T6 can be specifically configured to separate airways between the output port 530 and the converging node, when a pressure monitored by the pressure sensor exceeds a preset threshold. For example, the safety valve T6 is a three-way valve, wherein a first port of the three-way valve is connected with the output port 530, a second port of the three-way valve is connected with the converging node, and a third port of the three-way valve is the exhaust port. Under normal circumstances, the first port of the three-way valve is connected (communicated) with the second port of the three-way valve, while the first port of the three-way valve is disconnected from the exhaust port, and the second port of the three-way valve is disconnected from the exhaust port. The pressure sensor outputs the monitored pressure to the second ventilation control apparatus. The second ventilation control apparatus determines whether the pressure exceeds the preset threshold, and outputs a corresponding control signal to the three-way valve, when determining that the pressure exceeds the preset threshold. When triggered by the control signal, the three-way valve disconnects the first port from the second port and connects(communicates) the first port with the exhaust port, thereby releasing the gas with excessive pressure and improving safety. The preset threshold can be set based on clinical experience or doctor requirements.

The human-computer interaction apparatus of the anesthesia machine also includes a display. When the respiratory support mode, which is received by the human-computer interaction apparatus, is respiratory support which is periodic, the display is configured to output information which indicates to turn off the anesthetic delivery apparatus 20 or information which indicates to switch an anesthesia mode of the patient, so as to prevent the anesthetic from the anesthetic delivery apparatus 20 to be released into the environment rather than be provided to the patient after passing through the respiratory tract. When the respiratory support mode, which is received by the human-computer interaction apparatus, is respiratory support which is periodic, the display is configured to display a pressure waveform W (as shown in FIG. 9), which is convenient for the user to view and analyse. The pressure in the pressure waveform W is monitored by a pressure sensor.

In this embodiment, when the anesthetic delivery apparatus 20 is an electronic evaporation tank, the anesthesia machine is further configured to obtain a working state of the electronic evaporation tank. When the respiratory support mode, which is received by the human-computer interaction apparatus, is respiratory support which is periodic, the anesthesia machine is also configured to determine whether a current working state of the electronic evaporation tank is to stop working, that is, to determine whether the electronic evaporation tank is closed. Only after determining that the electronic evaporation tank is closed, the anesthesia machine is capable of switching to respiratory support which is periodic mode. Only after determining that the electronic evaporation tank is closed, the ventilation module 50 performs respiratory support which is periodic, which improves the safety of the anesthesia machine.

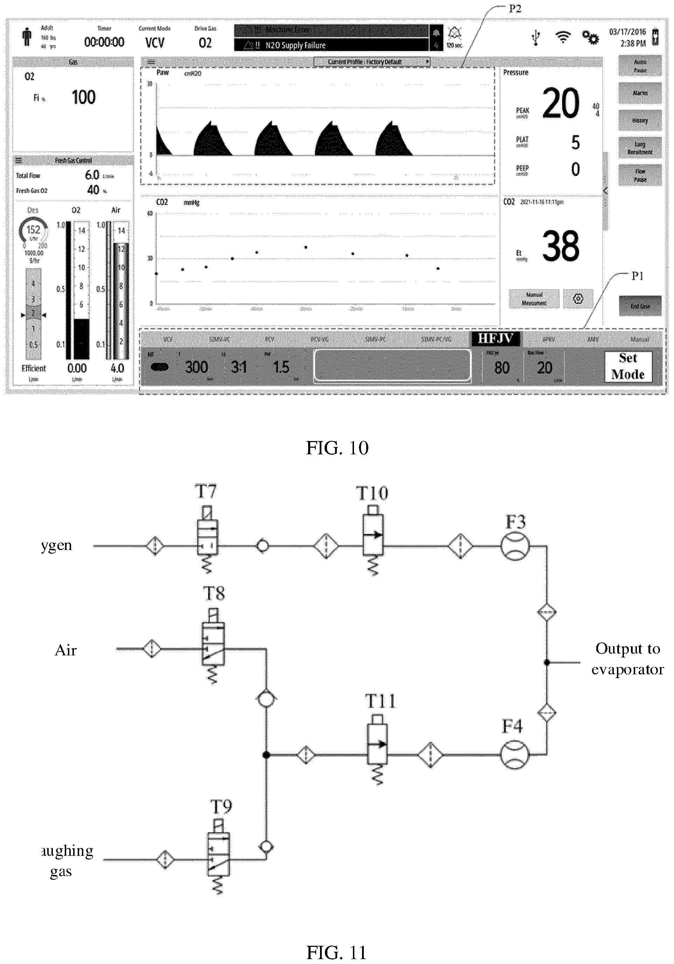

Further combined with FIG. 10, which is a diagram of a main monitoring interface on a display in an anesthesia machine provided by this disclosure. The main monitoring interface includes a switching area for a ventilation mode P1. High-frequency jet ventilation, as one of the ventilation modes, is presented in the switching area for a ventilation mode P1 in a form of a tab. When it is desired to use the anesthesia machine to provide respiratory support which is periodic, an "HFJV" icon in the switching area for a ventilation mode P1 is selected and a "SET MODE" icon is used to confirm, so as to complete the selection of respiratory support which is periodic mode. At this time, the ventilation module 50 can be controlled to start and provide periodic high-frequency jet ventilation to the patient. The pressure monitored during high-frequency jet ventilation is displayed in the pressure waveform area P2 of a main monitoring interface. When it is desired to use the anesthesia machine to provide anesthesia respiratory support which is periodic, other ventilation mode icons in the switching area for a ventilation mode P1 can be selected, such as a "VCV" icon. At this time, the first ventilation control apparatus 40 can control the respiratory loop to provide the periodic anesthetic respiratory support to the patient.

In some embodiments, when the human-computer interaction apparatus receives any respiratory support mode, instructions based on the same respiratory support mode may be transmitted to the first ventilation control apparatus and the second ventilation control apparatus, respectively. The two ventilation control apparatuses respectively start or stop the corresponding respiratory support mode according to the received instructions. For example, when the respiratory support mode received by the human-computer interaction apparatus is respiratory support which is periodic, the first ventilation control apparatus 40 controls the respiratory loop to stop outputting the second gas with anesthetic to the outside, and stops providing the anesthetic respiratory support which is periodic, to the patient, the second ventilation control apparatus synchronously controls the ventilation module 50 (for example, the gas delivery branch) to start periodically providing the first gas to the patient and perform high-frequency/jet ventilation.

In the above embodiments, the second ventilation control apparatus and the first ventilation control apparatus 40 are two independent ventilation control apparatuses. Of course, in other embodiments, the second ventilation control apparatus and the first ventilation control apparatus 40 may also be the same ventilation control apparatus, i.e., the control function of the ventilation module 50, may be further integrated with that of the first ventilation control apparatus. That is, the first ventilation control apparatus also has the function of the above-mentioned second ventilation control apparatus, and is configured to control the gas delivery branch to provide respiratory support which is periodic to the patient. In specific operations, after the human-computer interaction apparatus receives an instruction to select a respiratory support mode and transmits it to the first ventilation control apparatus, the first ventilation control apparatus determines whether to control the respiratory loop to provide anesthesia respiratory support which is periodic, and/or control the gas delivery branch to provide respiratory support which is periodic, to the patient.

As shown in FIGS. 6-8, the pressure sensor may be disposed between the second control valve T5 and the output port 530. The safety valve T6 is also provided between the second control valve T5 and the output port 530.

Multiple pressure sensors may be provided. In this embodiment, the first pressure sensor P1 and the second pressure sensor P2 are provided as an example for description. The pressure monitored by the first pressure sensor P1 is configured to trigger the safety valve T6, and the pressure monitored by the second pressure sensor P2 is configured to display the waveform.

Specifically, the first pressure sensor P1 is close to the safety valve T6. The first pressure sensor P1 monitors the pressure of the high-frequency gas. When it is found that the pressure value monitored by the first pressure sensor P1 is abnormal, the safety valve T6 is switched to separate the patient-side airway from the airway of the ventilation module 50. At the same time, the patient-side gas can be discharged through the safety valve T6, so as to prevent barotrauma at the patient side.

The second pressure sensor P2 is close to the patient end, that is, close to the output port 530. A pressure sampling port is provided at the patient end, which is connected with the second pressure sensor P2 of the ventilation module 50 through a pressure sampling tube. Therefore, the display displays, on the interface, a pressure oscillation waveform W, which is sampled by the second pressure sensor P2. Of course, in some embodiments, the action of the safety valve T6 can also be triggered based on the second pressure sensor P2, which is adjacent the patient end, and data of the pressure waveform W, which is displayed on the display, can also be sampled by the first pressure sensor P1, which is adjacent to the safety valve T6.

The ventilation module 50 can perform high-frequency/jet ventilation by controlling the switching frequency of the second control valve T5. The specific process of high-frequency/jet ventilation has been described in detail in the above content. The ventilation module 50 regulates the frequency of high-frequency/jet ventilation (i.e., the gas delivery frequency) of the second control valve T5 to achieve ventilation with different ventilation frequencies. For example, when the ventilation module 50 implements jet ventilation, the ventilation frequency of the jet ventilation apparatus can be lowered to form a low-frequency ventilation apparatus, and the low-frequency ventilation can be achieved with corresponding accessories (such as nasal plugs, nasal masks, masks, etc.) .

As shown in FIGS. 3 and 4, the anesthesia machine also includes a monitoring apparatus for flow amount 60. The monitoring apparatus for flow amount 60 is configured to control (regulate) the flow amount of the first gas. It can be mechanical or fully electronic. The monitoring apparatus for flow amount 60 can also be configured to detect the flow amount of the first gas. There are various connection methods between the monitoring apparatus for flow amount 60, the anesthetic delivery apparatus 20 and the ventilation module 50. For example, in the embodiment shown in FIG. 3, the gas source module 10 is connected with the anesthetic delivery apparatus 20 through the monitoring apparatus for flow amount 60, wherein the ventilation module 50 is connected with the gas source module 10. In FIG. 3 specifically, the gas source interface is connected with the evaporator 210 through the monitoring apparatus for flow amount 60. For another example, in the embodiment shown in FIG. 4, the anesthesia machine also includes a three-way valve T12. The three-way valve T12 includes a first port 1 which is connected with the monitoring apparatus for flow amount 60, a second port 2 which is connected with the anesthetic delivery apparatus 20, and a third port 3 which is connected with the ventilation module 50. That is, the gas source module 10 is connected with an input end of the monitoring apparatus for flow amount 60. An output end of the monitoring apparatus for flow amount 60 can be connected with the anesthetic delivery apparatus 20 under the control of the three-way valve T12, or can be connected with the ventilation module 50 under the control of the three-way valve T12, that is, the three-way valve T12 is configured to enable the monitoring apparatus for flow amount 60 to connect with one of the anesthetic delivery apparatus 20 and the ventilation module 50.

The gas source module 10 may also include a laughing gas unit which provides a laughing gas. The laughing gas unit may be a laughing gas interface, which is connected with an external laughing gas source, such as a laughing gas pipeline in a hospital, a laughing gas bottle, etc. The monitoring apparatus for flow amount 60 may have three input interfaces, which are respectively connected with the oxygen interface, the air interface, and the laughing gas interface. FIG. 11 corresponds to the monitoring apparatus for flow amount 60 in the embodiment in FIG. 3. The monitoring apparatus for flow amount 60 includes a third switch valve T7, a fourth switch valve T8, a fifth switch valve T9, a fourth control valve T10, a fifth control valve T11, a third flow sensor F3 and a fourth flow sensor F4. One end of the third switch valve T7 is connected with the oxygen interface, and the other end of the third switch valve T7 is connected with the input end of the third flow sensor F3 through the fourth control valve T10. One end of the fourth switch valve T8 is connected with the air interface, and one end of the fifth switch valve T9 is connected with the laughing gas interface. The other end of the fourth switch valve T8 and the other end of the fifth switch valve T9 are converged with each other and then connected with the input end of the fourth flow sensor F4 through the fifth control valve T11. The output end of the third flow sensor F3 and the output end of the fourth flow sensor F4 are converged with each other and then connected with the evaporator 210. The third switch valve T7, the fourth switch valve T8 and the fifth switch valve T9 are all configured to control a connection/disconnection state of the gas path. The fourth control valve T10 and the fifth control valve T11 are used for flow amount control.

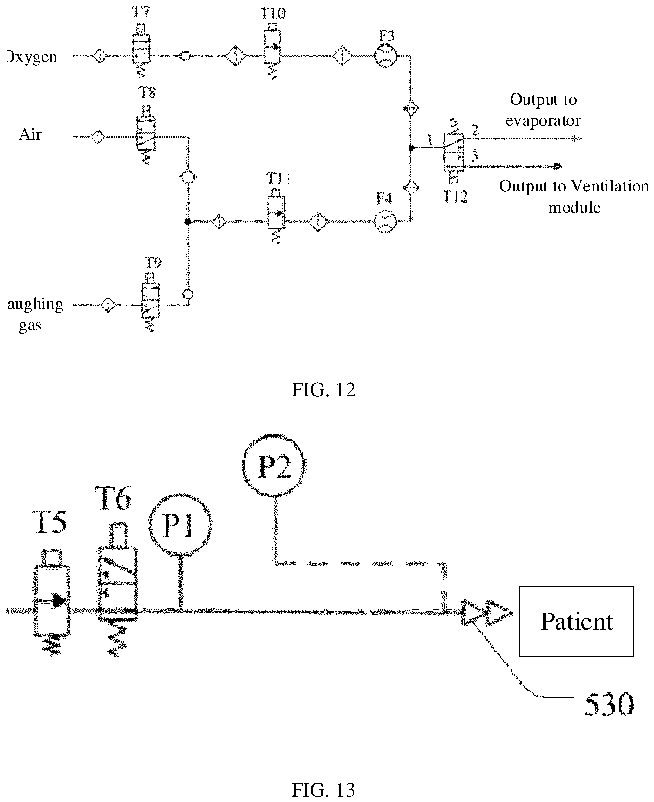

FIG. 12 corresponds to the monitoring apparatus for flow amount 60 of the embodiment in FIG. 4. The output end of the third flow sensor F3 and the output end of the fourth flow sensor F4 are converged with each other and then connected with the input port 1 of the three-way valve T12. The three-way valve T12 has a first output port 2 and a second output port 3. The first output port 2 of the three-way valve T12 is connected with the evaporator 210, and the second output port 3 of the three-way valve T12 is connected with the ventilation module (such as a high-frequency ventilation apparatus 501). The three-way valve T12 has two states, one state is that its input port 1 is connected with the first output port2, and the other state is that its input port 1 is connected with the second output port 3. That is, by controlling the three-way valve T12, it is possible to select whether the gas is outputted to the evaporator 210 or the high-frequency ventilation apparatus 501. The third switch valve T7, the fourth switch valve T8 and the fifth switch valve T9 are all configured to control a connection/disconnection state of the gas path. The fourth control valve T10 and the fifth control valve T11 are used for flow amount control.

In an embodiment shown in FIG. 4 , the ventilation module 50 may include a gas delivery branch and an output port, but the second control valve T5 is not required in the gas delivery branch. Instead, the gas delivery branch and the monitoring apparatus for flow amount 60 share the fourth control valve T10 and the fifth control valve T11, wherein the fourth control valve T10 and the fifth control valve T11 are all used for periodic ventilation control, that is, the functions of the fourth control valve T10 and the fifth control valve T11 can be the same as the above-mentioned second control valve. For example, both the fourth control valve T10 and the fifth control valve T11 can be controlled to switch an amount of flow at a certain frequency, so as to enable a pulse frequency of the gas flow at the first port 1 of the three-way valve T12 to be greater than or equal to 3Hz, or between 50-1500bpm. Specifically, taking FIG. 12 as an example, in an alternative embodiment, the first port 1 of the three-way valve T12 is connected with the monitoring apparatus for flow amount 60, the second port 2 of the three-way valve T12 is connected with the anesthetic delivery apparatus 20, the third port 3 of the three-way valve T12 is connected with the ventilation module 50. When the respiratory support mode received by the anesthesia machine is respiratory support which is periodic, the first port 1 and the third port 3 of the three-way valve T12 are connected, and the fourth control valve T10 and the fifth control valve T11 are connected with the ventilation module 50, so as to switch the flow amounts according to a preset frequency, thus enabling the ventilation module 50 to ventilate the patient at the first ventilation frequency. The first ventilation frequency may be, for example, greater than or equal to 3 Hz, or between 50-1500 bpm. In this way, by switching opening degrees of the two control valves T10 and T11, the oxygen-air mixed gas (it can also be a mixed gas of oxygen, air and laughing gas) forms a periodic gas flow. This gas flow with high periodicity is transmitted along the gas delivery branch to the output port. If the output port 530 of the ventilation module 50 is connected with the jet accessory, the periodic gas flow passes through the jet accessory and then provides jet ventilation to the patient. When the respiratory support mode received by the anesthesia machine is anesthesia respiratory support which is periodic, the first port 1 and the second port 2 of the three-way valve T12 are connected, the fourth control valve T10 and the fifth control valve T11 are connected with the anesthetic delivery apparatus 20 for providing the first gas to the anesthetic delivery apparatus at a second ventilation frequency, so as to enable the first gas to be mixed with the anesthetic at the second ventilation frequency for obtaining the second gas and ventilating the patient by the respiratory loop. The first ventilation frequency is higher than the second ventilation frequency. The second ventilation frequency may be zero, that is, the fourth control valve T10 and the fifth control valve T11 can be opened.

It can be understood that in the embodiment shown in FIG. 4, gas can be introduced from a rear end of the monitoring apparatus for flow amount 60 to the ventilation module. Compared with the solution in FIG. 3, the ventilation module in the solution in FIG. 4 borrows (shares) some components (T10 and T11) of the monitoring apparatus for flow amount 60, which greatly simplifies the structure of the ventilation module.

It can be seen from the above that, the first gas is at least oxygen-containing gas, and the anesthesia machine may also include an auxiliary gas supply module, which is configured to receive the first gas, which is outputted by the gas source module 10, and to use the first gas to provide respiratory support to the patient at a flow amount of 20-80 litres/minute. Respiratory support provided by the auxiliary gas delivery module is non-respiratory support which is periodic. Non-respiratory support which is periodic refers to a continuous delivery of gas to the patient. This manner is actually oxygen therapy of high-flow amount. In this way, the anesthesia machine can provide the oxygen therapy of high-flow amount to patients when anesthesia is not needed, and has a wide range of applications.

In some embodiments, the anesthesia machine may include a main frame having a housing, and at least part of the ventilation module may be arranged inside an accommodation space formed by the housing, forming an integrated whole with the anesthesia machine. That is, at least part of the ventilation module is integrated inside the anesthesia machine and integrated with the anesthesia machine. For example, the second ventilation control apparatus, which is included in the ventilation module, is built into the main frame and is in communicative connection with the human-computer interaction apparatus of the anesthesia machine.

The anesthesia machine of this disclosure, as integrated with the ventilation module, can provide following ventilation control: when the received respiratory support mode is anesthesia respiratory support which is periodic, controlling the first gas to mix with an anesthetic, so as to obtain a second gas, controlling the second gas to enter into a respiratory loop of the anesthesia machine, and controlling the respiratory loop to periodically output the second gas, so as to provide anesthesia respiratory support which is periodic; when the received respiratory support mode is respiratory support which is periodic, controlling the first gas to enter into a ventilation module of the anesthesia machine, and controlling the ventilation module to periodically output the first gas, so as to provide respiratory support which is periodic; wherein respiratory support which is periodic includes no gas delivery to the patient for at least a period of time in a respiratory cycle. For example, when the user wants to provide jet ventilation through the anesthesia machine, the ventilation module can be controlled to output periodic first gas at a certain frequency. The periodic first gas can complete jet ventilation after passing through the jet accessory.

This disclosure creatively integrates the jet Ventilation (JV) function into a general anesthesia machine, so as to support, by direct use of the anesthesia machine, applications, such as pharyngeal, tracheal, bronchial or lung surgeries where tracheal intubation ventilation is not applicable. For example, when using the anesthesia machine provided by this disclosure, the doctor puts the jet needle or nozzle into a suitable position in the pharynx or trachea, and uses the anesthesia machine to perform jet ventilation and respiratory support. On the one hand, the diameter of the jet needle or nozzle is very small, leaving enough space for the doctor to observe and perform laryngoscopy surgery. On the other hand, due to the characteristics of jet ventilation, especially the characteristics of high-frequency (of 3Hz or above, or 50-1500bpm) jet ventilation, a pulmonary pressure of the patient changes very little between inhalation and exhalation, so that no large fluctuations are caused in the pulmonary respiration, which undoubtedly provides convenience for one-lung ventilation or a pulmonary surgery. It is easy to understand that because jet ventilation uses a jet needle and a nozzle for ventilation, no sealed connection is formed between the airway system of the anesthesia machine and the respiratory system of the patient. Therefore, this type of ventilation cannot simultaneously inject aesthetics through the jet needle and nozzle. Otherwise, the anesthetic may leak out. In this case, the anesthesia needs to be administered intravenously.

This disclosure integrates ventilation modules, such as jet ventilation functions, into a general anesthesia machine, and integrates board cards, display screens, etc. into one device, which brings convenience to the management of surgery device and allows more hospitals to perform pharyngeal surgery. It has great clinical value for anesthesia surgeries that cannot be performed with tracheal intubation and ventilation, such as tracheal, bronchial or lung surgeries.

The description has been made with reference to various exemplary embodiments herein. However, those skilled in the art recognize that changes and modifications can be made to the exemplary embodiments without departing from the scope herein. For example, various operation steps and components for performing operation steps may be implemented in different ways according to a specific application or considering any number of cost functions associated with the operation of the system (for example, one or more steps may be deleted, modified, or incorporated into other steps).

In addition, as understood by those skilled in the art, the principles herein may be reflected in a computer program product on a computer-readable storage medium that is pre-installed with computer-readable program codes. Any tangible, non-transitory computer-readable storage medium can be used, including magnetic storage devices (hard disks, floppy disks, etc.), optical storage devices (CD-ROM, DVD, Blu Ray disks, etc.), flash memory, and/or the like. These computer program instructions can be loaded onto a general-purpose computer, a dedicated computer, or other programmable data processing device to form a machine, so that these instructions executed on a computer or other programmable data processing apparatus can generate an apparatus that implements a specified function. These computer program instructions can also be stored in a computer-readable memory that can instruct a computer or other programmable data processing device to operate in a specific manner, so that the instructions stored in the computer-readable memory can form a manufactured product, including an implementation apparatus that implements a specified function. The computer program instructions can also be loaded onto a computer or other programmable data processing device, so that a series of operating steps are performed on the computer or other programmable device to produce a computer-implemented process, so that the instructions executed on a computer or other programmable data processing device can provide steps for implementing specified functions.

Although the principles herein have been shown in various embodiments, many modifications of structures, arrangements, proportions, elements, materials, and components particularly suitable for the specific environmental and operational requirements may be used without departing from the principles and scope of the present disclosure. The above modifications and other changes or amendments are included in the scope of this disclosure.

The foregoing specific description has been described with reference to various embodiments. However, those skilled in the art recognize that various modifications and changes can be made without departing from the scope of the present disclosure. Therefore, consideration of the present disclosure is in an illustrative rather than a restrictive sense, and all such modifications are included within the scope thereof. Also, the advantages of various embodiments, other advantages, and the solutions to problems have been described above. However, the benefits, advantages, solutions to problems, and any elements that can produce these, or solutions that make them more explicit, should not be interpreted as critical, necessary, or essential. The term "comprising", and any other variants thereof used herein are non-exclusive, so that the process, method, document, or device that includes a list of elements includes not only these elements, but also other elements that are not explicitly listed or do not belong to the process, method, system, document, or device. Furthermore, the term "coupling" and any other variations thereof used herein refer to physical connection, electrical connection, magnetic connection, optical connection, communicational connection, functional connection, and/or any other connection.

It should be noted that a person of ordinary skill in the art could also make several variations and improvements without departing from the concept of this disclosure, and these variations and improvements would all fall within the scope of protection of this disclosure. Therefore, the protection scope of this disclosure should be in accordance with the appended claims.

## Claims

1. An anesthesia machine, comprising a monitoring apparatus for flow amount, an anesthetic delivery apparatus which is connected with the monitoring apparatus for flow amount, a respiratory loop, a first ventilation control apparatus and a human-computer interaction apparatus:
the monitoring apparatus for flow amount is configured to regulate a flow amount of a first gas;
the anesthetic delivery apparatus is configured to mix said first gas with an anesthetic for obtaining a second gas, and deliver the second gas to the respiratory loop;
the respiratory loop is configured to deliver the second gas to a patient;
the first ventilation control apparatus is configured to control the respiratory loop to deliver the second gas to said patient, so as to provide for said patient anesthesia respiratory support which is periodic;
the human-computer interaction apparatus is configured to receive, from a user, a respiratory support mode which is provided for a patient by the anesthesia machine;
**characterized in that**, the anesthesia machine further comprises:
a ventilation module, which is configured to receive a first gas and provide, by using said first gas, for said patient respiratory support which is periodic;
wherein the anesthesia machine is further configured to enable the first ventilation control apparatus to provide for said patient anesthesia respiratory support which is periodic, and/or enable the ventilation module to provide for said patient respiratory support which is periodic, according to the respiratory support mode, which is received by the human-computer interaction apparatus; wherein respiratory support which is periodic comprises no gas delivery to a patient for at least a period of time in a respiratory cycle.

2. The anesthesia machine according to claim 1, **characterized in that**, the ventilation module comprises a high-frequency ventilation apparatus, which is configured to ventilate a patient at a ventilation frequency of at least 3 Hz, so as to provide for said patient respiratory support which is periodic.

3. The anesthesia machine according to claim 2, **characterized in that**, the high-frequency ventilation apparatus comprises a gas delivery branch, a second control valve and an output port; wherein the high-frequency ventilation apparatus is configured to receive, through the gas delivery branch, said first gas; the second control valve is arranged inside the gas delivery branch, and is controlled to switch an amount of flow at a switching frequency of at least 3 Hz, so as to enable said first gas, which is outputted from the output port, to form a high-frequency gas flow.

4. The anesthesia machine according to claim 2, **characterized in that**, further comprising a jet accessory, which is connected with the high-frequency ventilation apparatus, wherein the jet accessory is configured to receive said first gas, which is outputted by the high-frequency ventilation apparatus, and enable said first gas to be outputted in a gas flow of high-frequency jet.

5. The anesthesia machine according to any one of claims 1-4, **characterized in that**, further comprising a gas source module which provides the first gas, wherein the gas source module is connected with the anesthetic delivery apparatus through the monitoring apparatus for flow amount, and the ventilation module is connected with the gas source module.

6. The anesthesia machine according to any one of claims 1-5, **characterized in that**, the gas source module comprises an oxygen unit, and the first gas comprises oxygen, which is provided by the oxygen unit;
the ventilation module comprises an oxygen branch, a second control valve and an output port; wherein the oxygen unit is connected with the output port through the oxygen branch, the second control valve is arranged inside the oxygen branch, so as to control a flow amount of oxygen inside the oxygen branch and enable said oxygen, which is outputted by the output port, to form a high-frequency gas flow.

7. The anesthesia machine according to any one of claims 1-5, **characterized in that**, the gas source module comprises an air unit, and the first gas comprises air, which is provided by the air unit;
the ventilation module comprises an air branch, a second control valve and an output port; wherein the air unit is connected with the output port through the air branch, the second control valve is arranged inside the air branch, so as to control a flow amount of air inside the air branch and enable said air, which is outputted by the output port, to form a high-frequency gas flow.

8. The anesthesia machine according to any one of claims 1-5, **characterized in that**, the gas source module comprises: an oxygen unit which is configured to provide oxygen, and an air unit which is configured to provide air; and the first gas comprises: the oxygen which is provided by the oxygen unit, and the air which is provided by the air unit;
the ventilation module comprises an oxygen branch, an air branch and an output port; wherein the oxygen branch is connected with the oxygen unit, the air branch is connected with the air unit; the oxygen branch and the air branch are converged with each other and then connected with the output port; wherein a first control valve is arranged inside the oxygen branch, so as to control a flow amount of oxygen inside the oxygen branch and enable said oxygen inside the oxygen branch to form a high-frequency gas flow; a third control valve is arranged inside the air branch, so as to control a flow amount of air inside the air branch and enable said air inside the air branch to form a high-frequency gas flow.

9. The anesthesia machine according to any one of claims 1-5, **characterized in that**, the gas source module comprises: an oxygen unit which is configured to provide oxygen, and an air unit which is configured to provide air; and the first gas comprises: the oxygen which is provided by the oxygen unit, and the air which is provided by the air unit;
the ventilation module comprises an oxygen branch, an air branch, a second control valve and an output port; wherein the oxygen branch is connected with the oxygen unit, the air branch is connected with the air unit, the oxygen branch and the air branch are converged with each other and then connected with the output port through the second control valve; wherein a first control valve is arranged inside the oxygen branch, a third control valve is arranged inside the air branch, wherein the first control valve is configured to control a flow amount of oxygen inside the oxygen branch, the third control valve is configured to control a flow amount of air inside the air branch, the second control valve is configured to enable a mixed gas, which is formed after converging said air and said oxygen, to form a high-frequency gas flow.

10. The anesthesia machine according to any one of claims 6-9, **characterized in that**, a ventilation frequency of the high-frequency gas flow, which flow is formed at the output port, is at least 3 Hz.

11. The anesthesia machine according to any one of claims 6-9, **characterized in that**, the output port is connected with a jet accessory, and the high-frequency gas flow, which is outputted by the output port, forms a gas flow of high-frequency jet after passing through the jet accessory.

12. The anesthesia machine according to claim 11, **characterized in that**, a ventilation frequency of the gas flow of high-frequency jet is 50-1500 bpm.

13. The anesthesia machine according to any one of claims 6-7, or 9, **characterized in that**, a pressure sensor, which is configured to monitor a pressure, is provided between the second control valve and the output port;
the human-computer interaction apparatus comprises a display; wherein the display is configured to display waveform(s) of the pressure, wherein the pressure is monitored by the pressure sensor, when the respiratory support mode, which is received by the human-computer interaction apparatus, is respiratory support which is periodic.

14. The anesthesia machine according to claim 13, **characterized in that**, a safety valve, which is configured to release the pressure, is further provided between the second control valve and the output port.

15. The anesthesia machine according to claim 1, **characterized in that**, the ventilation module comprises a gas delivery branch, a second control valve and an output port; wherein the second control valve is arranged inside the gas delivery branch, so as to control the ventilation module to provide respiratory support which is periodic; wherein the ventilation module is further configured to receive, through the gas delivery branch, said first gas; wherein said first gas is outputted through a jet accessory, which is connected with the output port, so as to form a jet gas flow.

16. The anesthesia machine according to claim 1, **characterized in that**, further comprising a jet accessory, which is connected with the ventilation module, wherein the jet accessory is configured to receive said first gas, which is outputted by the ventilation module, and enable said first gas to form a jet gas flow.

17. The anesthesia machine according to claim 16, **characterized in that**, the jet accessory comprises an jet channel, wherein an output aperture of the jet channel is less than 4 mm.

18. The anesthesia machine according to claim 5, **characterized in that**, the gas source module comprises an oxygen unit, and the first gas comprises oxygen, which is provided by the oxygen unit;
the ventilation module comprises an oxygen branch, a second control valve and an output port; wherein the oxygen unit is connected with the output port through the oxygen branch, the output port is connected with a jet accessory, the second control valve is arranged inside the oxygen branch, so as to control a flow amount of oxygen inside the oxygen branch and enable said oxygen, which is outputted by the output port, to form a periodic jet flow after passing through the jet accessory.

19. The anesthesia machine according to claim 5, **characterized in that**, the gas source module comprises an air unit, and the first gas comprises air, which is provided by the air unit;
the ventilation module comprises an air branch, a second control valve and an output port; wherein the air unit is connected with the output port through the air branch, the output port is connected with a jet accessory, the second control valve is arranged inside the air branch, so as to control a flow amount of air inside the air branch and enable said air, which is outputted by the output port, to form a periodic jet flow after passing through the jet accessory.

20. The anesthesia machine according to claim 5, **characterized in that**, the gas source module comprises: an oxygen unit which is configured to provide oxygen, and an air unit which is configured to provide air; and the first gas comprises: the oxygen which is provided by the oxygen unit, and the air which is provided by the air unit;
the ventilation module comprises an oxygen branch, an air branch and an output port; wherein the oxygen branch is connected with the oxygen unit, the air branch is connected with the air unit, the oxygen branch and the air branch are converged with each other and then connected with the output port, wherein the output port is connected with a jet accessory; wherein a first control valve is arranged inside the oxygen branch, so as to control a flow amount of oxygen inside the oxygen branch and enable said oxygen inside the oxygen branch to form a periodic gas flow; a third control valve is arranged inside the air branch, so as to control a flow amount of air inside the air branch and enable said air inside the air branch to form a periodic gas flow;
a periodic jet gas flow is formed, when a mixed gas, which is formed after converging the periodic gas flow of the oxygen branch and the periodic gas flow of the air branch, is outputted through the jet accessory, which accessory is connected with the output port.

21. The anesthesia machine according to claim 5, **characterized in that**, the gas source module comprises: an oxygen unit which is configured to provide oxygen, and an air unit which is configured to provide air; and the first gas comprises: the oxygen which is provided by the oxygen unit, and the air which is provided by the air unit;
the ventilation module comprises an oxygen branch, an air branch, a second control valve and an output port; wherein the oxygen branch is connected with the oxygen unit, the air branch is connected with the air unit, the oxygen branch and the air branch are converged with each other and then connected with the output port through the second control valve, wherein the output port is connected with a jet accessory; wherein a first control valve is arranged inside the oxygen branch, a third control valve is arranged inside the air branch, wherein the first control valve is configured to control a flow amount of oxygen inside the oxygen branch, the third control valve is configured to control a flow amount of air inside the air branch, the second control valve is configured to enable a mixed gas, which is formed after converging said air and said oxygen, to form a periodic jet gas after passing through the jet accessory, which is connected with the output port.

22. The anesthesia machine according to claim 1, **characterized in that**, the ventilation module comprises a second ventilation control apparatus and a gas delivery branch; wherein the second ventilation control apparatus is configured to control the gas delivery branch to provide for a patient respiratory support which is periodic.

23. The anesthesia machine according to claim 1, **characterized in that**, the ventilation module comprises a gas delivery branch, wherein the first ventilation control apparatus is further configured to control the gas delivery branch to provide for said patient respiratory support which is periodic.

24. The anesthesia machine according to claim 1, **characterized in that**, the human-computer interaction apparatus comprises a display; wherein the display is configured to output information, which indicates to close the anesthetic delivery apparatus or to switch an anesthesia mode for a patient, when the respiratory support mode, which is received by the human-computer interaction apparatus, is respiratory support which is periodic.

25. The anesthesia machine according to claim 1, **characterized in that**, the anesthetic delivery apparatus is an electronic evaporation tank;
wherein the anesthesia machine is further configured to determine whether the electronic evaporation tank is closed and enable the anesthesia machine to provide respiratory support which is periodic after determining that the electronic evaporation tank is closed, when the respiratory support mode, which is received by the human-computer interaction apparatus, is respiratory support which is periodic.

26. The anesthesia machine according to claim 1, **characterized in that**, further comprising a three-way valve, wherein the three-way valve comprises a first port which is connected with the monitoring apparatus for flow amount, a second port which is connected with the anesthetic delivery apparatus, and a third port which is connected with the ventilation module;
wherein the monitoring apparatus for flow amount comprises a fourth control valve and a fifth control valve; the first port and the third port are connected with each other, and the fourth control valve and the fifth control valve are connected with the ventilation module, so as to enable the ventilation module to ventilate a patient at a first ventilation frequency, when the respiratory support mode, which is received by the anesthesia machine, is respiratory support which is periodic;
the first port and the second port are connected with each other, and the fourth control valve and the fifth control valve are connected with the anesthetic delivery apparatus, so as to mix the first gas with the anesthetic at a second ventilation frequency for obtaining the second gas and enable the respiratory loop to ventilate a patient; wherein the first ventilation frequency is higher than the second ventilation frequency.

27. The anesthesia machine according to claim 1, **characterized in that**, the first gas is at least an oxygen-containing gas, and the anesthesia machine further comprises an auxiliary gas supply module, which is configured to receive the first gas and use the first gas to provide respiratory support to a patient at a flow amount of 20-80 liters/minute.

28. The anesthesia machine according to any one of claims 1-27, **characterized in that**, the ventilation module provides respiratory support which is periodic at a first ventilation frequency, the first ventilation control apparatus is further configured to control the respiratory loop to provide, at a third ventilation frequency, anesthesia respiratory support which is periodic; wherein the first ventilation frequency is higher than the third ventilation frequency.

29. The anesthesia machine according to any one of claims 3, 6, 7, 9, 13, 14, 15, 18, 19 and 21, **characterized in that**, the second control valve is selected from a group consisting of: an electromagnetic valve, an electromagnetic servo valve, an electromagnetic switch valve, and an electromagnetic proportional valve.

30. An anesthesia machine, **characterized in that**, comprising a gas source interface, an anesthetic delivery apparatus which is connected with the gas source interface, a respiratory loop, and a first ventilation control apparatus;
the gas source interface is connected with an external gas source;
the anesthetic delivery apparatus is configured to mix a gas, which is provided by the external gas source, with an anesthetic, and to deliver the mixed gas into the respiratory loop;
the first ventilation control apparatus is configured to control the respiratory loop to periodically deliver the mixed gas to a patient, so as to provide anesthesia respiratory support to said patient;
wherein the anesthesia machine further comprises:
a ventilation module, which is configured to provide respiratory support to a patient by using a gas, which is provided by the external gas source; wherein the ventilation module comprises a gas delivery branch, which is configured to receive said gas, which is provided by the external gas source, and provide respiratory support to said patient at a ventilation frequency of 3Hz or above.

31. The anesthesia machine according to claim 30, **characterized in that**, the ventilation module further comprises a second control valve, which is arranged inside the gas delivery branch, and an output port, which is connected with the gas delivery branch; wherein the output port is connected with a jet accessory, and the second control valve is controlled to switch an amount of flow at a switching frequency of at least 3 Hz, so as to enable a gas, which is outputted from the jet accessory, which accessory is connected with the output port, to form a gas flow of high-frequency jet.

32. An anesthesia machine, **characterized in that**, comprising a gas source interface, an anesthetic delivery apparatus which is connected with the gas source interface, a respiratory loop, and a first ventilation control apparatus;
the gas source interface is connected with an external gas source;
the anesthetic delivery apparatus is configured to mix a gas, which is provided by the external gas source, with an anesthetic, and deliver a mixed gas into the respiratory loop;
the first ventilation control apparatus is configured to control the respiratory loop to periodically deliver the mixed gas to a patient, so as to provide anesthesia respiratory support to the patient;
wherein the anesthesia machine further comprises:
a ventilation module, which is connected with a jet accessory and configured to receive and regulate a gas, which is outputted from the gas source interface, so as to enable said gas to provide respiratory support to a patient in a jet gas flow after passing through the jet accessory.

33. A ventilation control method for an anesthesia machine, **characterized in that**, comprising:
receiving a first gas;
receiving, from a user, a respiratory support mode which is provided by the anesthesia machine;
when the received respiratory support mode is anesthesia respiratory support which is periodic; controlling the first gas to be mixed with an anesthetic, so as to obtain a second gas, controlling the second gas to enter into a respiratory loop of the anesthesia machine, and controlling the respiratory loop to periodically output the second gas, so as to provide anesthesia respiratory support which is periodic;
when the received respiratory support mode is respiratory support which is periodic; controlling the first gas to enter into a ventilation module of the anesthesia machine, and controlling the ventilation module to periodically output the first gas, so as to provide respiratory support which is periodic; wherein respiratory support which is periodic comprises no gas delivery for at least a period of time in a respiratory cycle.
